# EUROPEAN PATENT SPECIFICATION

(11) **EP 1 021 196 B1**
(45) Date of publication and mention of the grant of the patent: **04.02.2004**
(21) Application number: 98946681.8
(22) Date of filing: 06.10.1998
(51) Int. Cl.: A61K 33/40, A61P 37/00, A61P 37/06

(54) **USE OF A CHEMICALLY-STABILIZED CHLORITE SOLUTION FOR INHIBITING AN ANTIGEN-SPECIFIC IMMUNE RESPONSE**
VERWENDUNG EINER CHEMISCH STABILISIERTEN CHLORITLÖSUNG ZUR INHIBITION DER ANTIGENSPEZIFISCHEN IMMUNANTWORT
UTILISATION DE SOLUTION DE CHLORITE STABILISEE PAR VOIE CHIMIQUE AUX FINS DE L'INHIBITION D'UNE REPONSE IMMUNITAIRE SPECIFIQUE D'UN ANTIGENE

(30) Priority: 06.10.1997 US 60953 P
(43) Date of publication of application: 26.07.2000
(73) Proprietor: OXO CHEMIE AG, 1700 Fribourg (CH)
(72) Inventor: KUHNE, Frederich W., Bangkok 10250 (TH); MCGRATH, Michael, Oxo Chemie, Inc., South San Francisco, CA 94080 (US); ENGLEMAN, Edgar G., Oxo Chemie, Inc., South San Francisco, CA 94080 (US)
(74) Representative: Olgemöller, Luitgard, Dr.
(86) International application number: PCT/IB1998/001676
(87) International publication number: WO 1999/017787

(56) References cited:
- EP-A- 0 200 157
- EP-A- 0 561 145
- WO-A-96/28173
- US-A- 4 851 222
- ELSTNER E.F.: "Oxygen radicals - Biochemical basis for their efficacy" KLINISCHE WOCHENSCHRIFT (KLIN. WOCHENSCHR.), 1991, 69/21-23 (949-956), XP002098533 Germany
- DATABASE AIDSLINE US NATIONAL LIBRARY OF MEDICINE (NLM),BETHESDA, MD, US BUSCH HW ET AL: "Treatment of HIV-infected patients with advanced symptomatic disease with WF10 solution (TCDO)." XP002098534 & INT CONF AIDS, AUG 7-12 1994, 10 (1) P204 (ABSTRACT NO. PB0245),
- DATABASE EMBASE ELSEVIER SCIENCE PUBLISHERS, AMSTERDAM, NL KEMPF S.R. ET AL: "Comparative study on the effects of chlorite oxygen reaction product TCDO (tetrachlorodecaoxygen) and sodium chlorite solution (NaClOinf 2) with equimolar chlorite content on bone marrow and peripheral blood of BDIX rats" XP002098535 & DRUGS UNDER EXPERIMENTAL AND CLINICAL RESEARCH (DRUGS EXP. CLIN. RES.), 1993, 19/4 (165-174), Switzerland

## Description

### Field of the Invention

The present invention relates to the use of a stabilized chlorite solution to inhibit antigen-specific immune responses. The stabilized chlorite solution inhibits antigen-specific immune responses by impeding antigen presentation by antigen presenting cells. The stabilized chlorite solution therefore is useful in treating diseases caused by or associated with unwanted or inappropriate antigen-specific immune responses including, for example, auto-immune diseases, hepatitis B and C, chronic hepatitis, chronic obstructive pulmonary disease, systemic lupus erythemotosus and in preventing rejection in organ transplant and graft patients (graft versus host disease). The stabilized chlorite solution also is useful in treating lymphoma, specifically, follicular non-Hodgkin's lymphoma.

### Background of the Invention

A feature common to an immune response is the recognition of an antigen (either foreign or self, but perceived as foreign), and subsequent processing by the immune system. Typically, antigen is enzymatically degraded in the cytoplasm, endoplasmic reticulum (ER) and lysosomes of cells, (usually macrophages, dendritic cells and other antigen presenting cells (APCs)), or in serum. The degraded antigen is presented on the surface of the APC by MHC class I or II molecules. This presentation of the antigenic epitope by the MHC molecule, and subsequent binding to the T cell receptor (TCR) of a T cell is known as antigen presentation. *See, for example:* Rodgers *et al*., CLINICAL IMMUNOLOGY, PRINCIPLES AND PRACTICE (RICH): "Antigens and antigen presentation," Chpt. 7, pp 114-131, Mosby, St. Louis, MO (1996); Roitt, ESSENTIAL IMMUNOLOGY, Blackwell Science, Oxford, England (1997).

T cells circulating in the body recognize and bind to an antigenic epitope (antigen) presented by the MHC (Class I or II) molecule through the TCRs on the surface of the T cell. Successful binding of the TCR to the presented antigenic epitope results in a cascade of events. For example, when T cells encounter antigen bound to MHC molecules on the surface of an APC, they can undergo profound phenotypic changes characterized by changes in gene expression, effector functions, secretion of lymphokines, and, under appropriate circumstances, cell proliferation. Inappropriate immune responses occur in a similar manner, however, and can lead to undesirable T cell proliferation, unwanted lymphokine secretion, and a state of autoimmunity.

In the course of a normal immune response the TCR must first be capable of recognizing and binding to the antigen presented. It is believed, however, that more than a simple binding of antigen is needed to bring about the cascade of events described above. Thus, it is thought that a ligand present on the APC must react with a costimulatory receptor on the T cell to bring about lymphocyte activation. Specifically, the B7 molecule on the surface of the APC interacts with its counterreceptor on the T cell, CD28, a molecule which forms a part of the TCR. Siegel, *et al*., CLINICAL IMMUNOLOGY, PRINCIPLES AND PRACTICE (RICH): "Signal Transduction and T lymphocyte activation," Chpt. 12, pp 192-216, Mosby, St. Louis, MO (1996). *See also* Roitt, *supra* at pp. 169-170.

One of the strongest immune responses is termed an allogeneic response, which involves the immune system reacting against non-self MHC alloantigens. This type of reactivity is observed, for example, in rejection of non-self grafts, such as transplanted organs, and clearly is undesirable in such situations. Reported mechanisms of immunosuppression that act by interfering with allorecognition (*i. e*. , by depletion of graft antigen, inhibition of APC function, blockade of surface receptor/co-receptor molecules, *etc*.) are ineffective for preventing or reducing the severity of an allogeneic response, however, because of their toxic side effects and their short-term activity. RICH *supra*., at "Concepts and challenges in solid organ transplantation," Chpt. 104, pp. 1593-1607. In addition, there are no reported treatment regimens that are effective in blocking the B7/CD28 co-stimulatory interaction.

The immune system of most mammals is capable of recognizing and responding to self and foreign antigens in an appropriate manner. The phenomenon where the immune system does not respond to self-antigens is termed immunological tolerance. Triplett, *J. Immunol*. **86:** 505-510, (1962). Tolerance to self antigens sometimes breaks down, however, causing autoimmunity, where T or B cells (or both), as well as various cytokines of a mammal, react against and destroy the antigens in the mammal's own tissues. In addition, mammals frequently show inappropriate immune responses to foreign antigens, causing an overstimulation or overactivation of the immune system that results in damage to normal, healthy tissue.

These autoimmune responses and inappropriate immune responses are responsible for a number of systemic immune diseases, including myasthenia gravis, systemic lupus erythematosus, serum disease, type I diabetes, rheumatoid arthritis, juvenile rheumatoid arthritis, rheumatic fever, Sjörgen syndrome, systemic sclerosis, spondylarthropathies, Lyme disease, sarcoidosis, autoimmune hemolysis, autoimmune hepatitis, autoimmune neutropenia, autoimmune polyglandular disease, autoimmune thyroid disease, multiple sclerosis, inflammatory bowel disease, colitis, Crohn's disease, chronic fatigue syndrome, and the like.

An important factor in autoimmune diseases is the presence of T cells directed against self tissue or antigens. When an antigen (or self-antigen) is presented by an APC, the T cells that possess these anti-self receptors bind to the presented antigenic epitope, and begin to differentiate and proliferate to eventually destroy the antigen (or self-antigen). Davis, *Annu. Rev. Biochem*., **59:475** (1990). Several mechanisms have been proposed to prevent anti-self T cells from differentiating. One mechanism is clonal anergy, which is the functional inactivation of a T cell. Schwartz in Rich, *supra*, "Mechanisms of Autoimmunity," Chpt. 69, pp 1053-61. The anergic T cell is unable to express IL-2, a cytokine necessary for T-cell proliferation. Accordingly, the T-cell cannot proliferate and is unable to cause symptoms of autoimmune disease.

Conventional methods of combatting autoimmune responses down- regulate the immune response by preventing or inhibiting T cell proliferation after antigen presentation. These methods attempt to inhibit formation and expansion of cytotoxic T cells after antigen presentation and release of cytokines (IL-1, IL-2, TNF, etc.). For example, cyclosporin A is known to prevent proliferation of T cells after antigen presentation by blocking production of IL-2. Methods of modulating the immune response that attempt to interfere with the production of stimulated T cells after antigen presentation characteristically require administration of a large quantity of therapeutic agent, which can cause undesirable toxic side effects.

Moreover, while expansion of anti-self T cells are necessary for some autoimmune diseases, their presence alone is not sufficient to cause all autoimmune responses. Schwartz, *supra*., at 1055. For example, polyclonal B cell activation is a common feature of systemic lupus erythematosus. Klinman, *et al., J. Exp. Med*., **165:1755** (1987). In addition, the presence of autoantibodies is not uncommon in organ-specific autoimmune diseases. Bernard *et al., Diabetes*, **41:40** (1992). Thus, preventing anti-self T cell proliferation alone may be ineffective in treating many autoimmune diseases.

There are instances other than autoimmune diseases where an immune response is not needed, or where it is desirable to suppress to some extent the immune response. Allergic responses to antigens and excessive inflammation are examples where the immune system has initiated an inappropriate immune response. Chronic viral infection with a hepatitis virus, such as hepatitis B or C is an example where excessive immunologic reactive inflammation causes end stage liver dysfunction and diseases such as cirrhosis and hepatoma. Rejection of transplanted organs and grafted tissue is another example. In addition, the transplanted organs or graft can sometimes elicit a graft vs. host response where the cells of the graft or organ mediate an immune response against healthy host cells.

In the case of organ transplants and tissue grafting, it is not advantageous to initiate an immune response to the foreign antigens of the transplanted or grafted organ. In these cases, the immune system must develop an immunological tolerance to the foreign antigens. In a similar manner, the immune system of the transplanted organ or graft also must develop a tolerance to host antigens. In the field of organ transplantation and grafting, the recipient's cellular immune response to the foreign graft can be depressed with cytotoxic agents that affect the lymphoid and other parts of the hematopoietic system. Graft acceptance is limited, however, by the tolerance of the recipient to these cytotoxic chemicals, many of which are similar to anticancer (antiproliferative) agents. Likewise, when using cytotoxic antimicrobial agents, particularly antiviral drugs, or when using cytotoxic drugs for autoimmune disease therapy, e.g., in treatment of systemic lupus erythematosis, one serious limitation is the toxic effects to the bone marrow and the hematopoietic cells of the body. A further limitation is the inability of the cytotoxic agents to induce an immunological tolerance to the foreign antigens.

Toxic side effects to normal tissues and cells also can limit the efficacy of most forms of nonsurgical cancer therapy, such as external irradiation and chemotherapy, because of the limited specificity of these treatment modalities for cancer cells. This limitation is also of importance when anti-cancer antibodies are used for targeting toxic agents, such as isotopes, drugs, and toxins, to cancer sites, because, as systemic agents, the antibodies also circulate to sensitive cellular compartments such as the bone marrow. In acute radiation injury, there is destruction of lymphoid and hematopoietic compartments which is a major factor in the development of septicemia and subsequent death.

Many different approaches have been undertaken to protect an organism from the side effects of radiation or toxic chemicals. One approach is to replace bone marrow cells after toxicity has developed. Another is to inject a chemical blocker which competes for the site of action of the toxic drug.

Neta *et al. (J. Immunol*. 136:2483-2485, 1986) showed that pre-treatment with recombinant interleukin-1 (IL-1) protects mice in a dose-dependent manner from the lethal effects of external beam irradiation, when the IL-1 was given 20 hr before irradiation. Other studies have shown the use of other cytokines in ameliorating the toxic side effects of radiation therapy and chemotherapy. Preventing secretion of cytokines and/or inhibiting antigen presentation in antigen presenting cells (macrophages, dendritic cells, etc.), however, has not been reported as useful (or not useful) in ameliorating these side effects.

Conventional immunosuppression also is ineffective in treating organ transplant and graft rejection. First, most immunosuppressive agents, such as antiproliferative and corticosteroids, display a low immunosuppressive efficacy. Second, excessive amounts of immunosuppressive agents, such as the monoclonal antibody OKT3, may produce toxic effects on T and B cells, leading to emergence of occult viral infections in, or neoplastic diseases of, lymphoid cells. Third, toxic effects on organs not belonging to the immune system result from administration of large doses of immunosuppressive agents such as cyclosporine.

Antigen presentation on APCs also has the effect of stimulating T helper cells to "help" B cells undergo proliferation and subsequent differentiation. After each division, B cells that bind antigen with higher affinity are allowed to divide again; those B cells whose immunoglobulin remain unmodified or have a lower affinity are allowed to die. B cells therefore initially proliferate, and then differentiate into plasma cells that secrete immunoglobulin as noted by the Ig subclasses. Typically, B cells secrete IgM first, followed by IgG, IgA and IgE. If B-cells continue to proliferate, but fail to differentiate, they could give rise to a lymphoproliferative disease, such as lymphoma. Gause, in Rich, *supra*, Ch. 113, pp 1745-1767.

Non-Hodgkin's follicular lymphoma (non-HIV) is one of the most common lymphomas in the United States. Approximately 40,000 new cases of lymphocytic lymphomas are diagnosed annually, with an estimated mortality of 19,000. Ries, *et al., Cancer Statistics Review 1973-1988*, National Institutes of Health Publ 91-2789, Washington, DC, 1991, National Cancer Institute. Follicular lymphoma, progresses relatively slowly over time and requires little therapy, except when it causes the patient discomfort or develops a life-threatening complication. Although falling in the low grade category of lymphoma, follicular lymphoma can not be cured given current therapeutic considerations, and is ultimately universally fatal.

In 1981, the first treatment of a patient with antiidiotypic antibody made from the patient's own B cell lymphoma was undertaken. Miller *et al., N. Engl. J. Med*., **306:517** (1982). More than 10 years ago, researchers used monoclonal anti-idiotypic antibodies for treatment of follicular lymphoma. This research found that lymphomas responded to anti-idiotype therapy in direct relationship to the proportion of T cells that co-existed within the lymphoma. These findings suggested that the malignant B cells somehow interacted with T cells and that the anti-idiotypic antibodies somehow changed either the growth conditions of the lymphoma cells or the T cell immune response against the B cells. Anti-idiotypic therapy has not been adopted, however, because, since the anti-idiotypic antibodies are made from the patient's own B cells (which have the inherent capacity to modify their structure), the B cell tumors also have the ability to somatically mutate their antigen binding site (*i.e*., idiotype) thus making them impervious to anti-idiotypic therapy. Gause, *supra* at Chpt. 113, pp 1745-1767).

More recently, dendritic cells incubated with lymphoma idiotypic-type (tumor-specific immunoglobulin) have been used to immunize patients against their own follicular lymphoma. Here, blood dendritic cells were removed from patients, incubated with their own tumor-specific antibocy, and injected back into the patient. A substantial number of patients responded by shrinkage of their tumors after injection thereby indicating that the dendritic cells induced a T cell response against the malignant B cells. These observations suggest that follicular lymphoma may be amenable to immunologic manipulation.

One of the pathogenic lesions within the follicular lymphoma process involves macrophage antigen processing and/or presentation. Despite the numerous treatment regimens for follicular lymphoma, and despite the recent advancements in cancer biotherapy trials, there have been no significant improvements in the management of lymphomas. *Id*., at 1763. Moreover, it has heretofore been unknown to treat lymphoma by regulating antigen presentation in APC.

Inhibiting an inappropriate immune response and inhibiting and/or preventing antigen presentation, while advantageous in ameliorating autoimmune disorders, allergic responses, transplant rejections, *etc.*, has the disadvantage of reducing the immune system's ability to fight off infections. Thus, known therapies for immunosuppression often are carried out in connection with administration of agents that stimulate phagocytic activity of phagocytic cells like macrophages, monocytes and polymorphomononuclear cells (PMNs) to fight off other infections. There are no known therapies capable of inhibiting an antigen-specific immune response, while at the same time stimulating phagocytic activity.

It has recently been postulated that an important component in the body's ability to control the duration and severity of the inflammatory response that accompanies macrophage activation during an immune response is the presence of macrophages that are "alternatively activated. " Stein *et al*., *(J. Exp. Med.* 176:287 (1992)). Unlike "classical" macrophage activation, which is induced by interferon-γ, TNF-α, IL-12, or bacterial lipopolysaccharide, the alternative pathway is induced by IL-4, IL-10, or IL-13, and is characterized by expression of the AMAC-1 gene, producing MIP-4 protein (macrophage inflammatory protein-4) and reduced secretion of proinflammatory cytokines. *See* Kodelja *et al., J. Immunol*. 160:1411 (1998); Schebesch *et al., Immunology* 92:478 (1997). Alternatively activated macrophages have been shown to actively inhibit mitogen-mediated lymphocyte proliferation. As such, the alternative pathway of macrophage activation is thought to act as an important modulator of the proinflammatory macrophage response. Indeed, it has been postulated that alternatively activated macrophages might play a key role in reducing inflammation in allergic and autoimmune diseases.

Aqueous solutions of a chemically stabilized chlorite solution that are capable of intravenous administration are known. Other chlorine-containing solutions also are known to have reported medicinal uses. For example, United States Patent No. 5,019,402 discloses a solution containing chlorine dioxide or a chlorine dioxide-liberating mixture of a chlorite, a weakly acidic buffer and a heat-activated saccharide which can be used for the sterilization *ex vivo* of stored blood components. Notably, however, the method is unsuitable for use with blood products containing red blood corpuscles, *i.e*., of leukocytes, blood platelets, coagulation factors and globulins. In whole blood, a corresponding disinfecting action does not occur, presumably because the red blood corpuscles are attacked more quickly by the chlorine dioxide than the targeted micro-organisms.

DE-OS 32 13 389, United States Patent No. 4,507,285 and United States Patent No. 4,296,103, describe chemically-stabilized chlorite matrices that are suitable for external or oral therapeutic use. Besides various bacterial infections, the external treatment of virus infections, such as herpes simplex and herpes zoster, may be possible in this manner. However, these documents do not report the use of these chlorite matrices for intravenous administration for inhibiting an antigen-specific immune response.

European Patent EP 0 200 157 and United States Patent No. 4,725,437 further describe solutions of a chemically-stabilized chlorite solution for intravenous and perioperative administration. The agent has proved to be effective in the treatment of *Candida albicans* infections. From EP 0 200 157, it is known to use such stabilized chlorite matrices for intravenous and/or local administration in cases of infectious conditions brought about by parasites, fungi, bacteria, viruses and/or mycoplasts. The action is thought to occur via phagocyte stimulation which is achieved by a single effective administration of the chlorite complex shortly after the infection. Down-regulation of an immune response and inhibition of antigen-specific immune responses are not described in these publications; rather, the postulated principle of action via phagocyte stimulation would lead to the opposite prediction.

It is apparent, therefore, that new methods of modifying the immune response are greatly to be desired. In particular, it is highly desirable to identify new methods of treating diseases associated with inappropriate antigen presentation, such as autoimmune disease, transplant rejection, and systemic lupus erythemotosus, and of treating diseases having symptoms of chronic inflammation due to inappropriate macrophage activation, such as hepatitis B and C, chronic hepatitis, and chronic obstructive pulmonary disease. It also is apparent that methods of treating lymphoproliferative diseases by preventing antigen presentation are desirable.

### Summary of the Invention

There exists a need to develop a method of inhibiting an antigen-specific immune response by inhibiting or preventing antigen presentation, while at the same time, stimulating phagocytic activity. It is therefore an object of the invention to provide a method of inhibiting an immune response by partially or completely blocking antigen presentation on antigen presenting cells. It is also an object of the present invention to inhibit the release of cytokines and proliferation of stimulated T cells by partially or completely blocking antigen presentation on antigen presenting cells. It is an additional object of the invention to provide a method of inhibiting an antigen-specific immune response, while at the same time stimulating phagocytic activity.

In accordance with these and other objects of the invention, there is provided a method of inhibiting an immune response comprising administering an inhibition effective amount of a stabilized chlorite solution containing an isotonic solution of 5 to 100 mMol of ClO₂⁻ per liter of solution. The method causes a partial or complete blockage of antigen presentation on antigen presenting cells including, *inter alia*, dendritic cells and macrophages.

In accordance with an additional object of the present invention, there is provided a method of inhibiting an inappropriate immune response comprising administering an inhibition effective amount of a chlorite solution containing an isotonic solution of 5 to 100 mMol of ClO₂⁻ per liter of solution. In accordance with yet another object of the invention, there is provided a method of treating an autoimmune disease comprising inhibiting antigen presentation in antigen presenting cells. This object can be achieved by administering to a mammal in need thereof, an inhibition effective amount of a chlorite solution containing an isotonic solution of 5 to 100 mMol of ClO₂⁻ per liter of solution.

In particular, there are provided methods of treating a disease selected from the group consisting of myasthenia gravis, systemic lupus erythematosus, serum disease, type I diabetes, rheumatoid arthritis, juvenile rheumatoid arthritis, rheumatic fever; Sjörgen syndrome, systemic sclerosis, spondylarthropathies, Lyme disease, sarcoidosis, autoimmune hemolysis, autoimmune hepatitis, autoimmune neutropenia, autoimmune polyglandular disease, autoimmune thyroid disease, multiple sclerosis, inflammatory bowel disease, colitis, Crohn's disease, and chronic fatigue syndrome.

In accordance with an additional object of the invention, there is provided a method of inhibiting transplant organ and graft rejection in a mammal, comprising inhibiting antigen presentation in antigen presenting cells. This object can be achieved by administering to a mammal in need thereof, an inhibition effective amount of a chlorite solution containing an isotonic solution of 5 to 100 mMol of ClO₂⁻ per liter of solution.

In accordance with another aspect of the invention there are provided methods of treating a disease selected from the group consisting of lymphoproliferative disease, hepatitis B, hepatitis C, chronic hepatitis, and chronic obstructive pulmonary disease, by administering to a patient suffering from the disease a therapeutically effective amount of an aqueous solution of a stabilized chlorite solution.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Brief Description of the Figures

Figure 1 illustrates the mechanism by which antigen presenting cells present antigens to activate T-cells and elicit an immune response or fail to present antigen resulting in an anergic response.
Figure 2 illustrates the effect of the chlorite solution of the invention in inhibiting proliferation of T cells from dendritic cells stimulated with allogeneic mixed leukocyte reaction.
Figure 3 illustrates the effect of the chlorite solution of the invention in inhibiting proliferation of T cells from monocytes stimulated with allogeneic mixed leukocyte reaction.
Figure 4 illustrates the effect of the chlorite solution of the invention in inhibiting soluble antigen-induced proliferation of T cells from dendritic cells.
Figure 5 illustrates the effect of the chlorite solution of the invention in inhibiting soluble antigen-induced proliferation of T cells from monocytes.
Figure 6 illustrates the relationship between the number of CD14⁺/CD69⁺ cells/µl over time in patients subjected to administration of WF-10.
Figure 7 illustrates the relationship between the number of CD14⁺/TNF cells/µl over time in patients subjected to adminstration of WF-10.
Figure 8 illustrates the relationship between the number of CD3⁺/CD8⁺/CD28⁻ cells/µl over time in patients subjected to administration of WF-10.
Figure 9 illustrates the relationship between the number of CD3⁺/CD8⁺ cells/µl over time in patients subjected to administration of WF-10.
Figure 10 illustrates the relationship between the phagocyte index in number of cells/µl over time in patients subjected to administration of WF-10.
Figure 11 illustrates the relationship between the CD14⁺/DR⁺ cells/µl over time in patients subjected to administration of WF-10.
Figure 12 illustrates the decline in antibody against double stranded DNA after treatment with WF-10 in a patient suffering from systemic lupus erythemotosus.

### Detailed Description of the Invention

The present invention provides methods of inhibiting antigen presentation in patients suffering from clinical conditions associated with inappropriate or excessive antigen presentation. The methods involve administering to the patient a therapeutically effective amount of a stabilized chlorite solution sufficient to inhibit antigen presentation and to alleviate symptoms associated with the clinical conditions. In particular, the methods of the invention are useful for preventing transplant rejection, and for treating autoimmune disease, systemic lupus erythematosus, lymphoproliferative disease such as lymphoma, and diseases associated with chronic inflammation. Diseases associated with chronic inflammation include chronic hepatitis, hepatitis B and C, chronic obstructive pulmonary disease, and all inflammation in mucosal disease (e.g. Crohn's disease and colitis).

The dosage of the stabilized chlorite preparation that is administered to a patient to achieve a desired therapeutic result will depend upon various factors, including the body weight and gender of the patient. Methods of adjusting dosage regimens to take body weight, gender, and other metabolic factors into account are well known in the art. The particular therapeutic endpoint that is to be achieved will vary depending upon the particular pathology and symptoms of the disease that is being treated, but these endpoints are well known in the art. For example, both hepatitis B and chronic persistent hepatitis are associated with laboratory findings of markedly elevated levels of transaminase activity. Efficacy of treatment using the chlorite preparation may be estimated by measuring levels of transaminase activity both before and after treatment. Similarly, patients suffering from systemic lupus erythemotosus display a high titer of antibodies against double-stranded DNA, and a reduction in this titer following treatment is one indication of the efficacy of the treatment. The skilled artisan readily will appreciate, however, that clinical benefit often may readily be ascertained by observing general improvement in the symptoms reported by a patient, without the need for a quantitative measurement of clinical response. Similarly, absence of a measurable response in certain laboratory findings does not of itself preclude the existence of clinically significant benefit.

In the context of the present invention, those skilled in the art will appreciate that the term "an inhibition effective amount" indicates an amount of solution which, when administered *in vivo* to a subject, will bring about an inhibition of the antigen presentation, and consequently, an inhibition of the proliferation of T cells. A therapeutically effective amount of the solution is that amount that produces a therapeutically significant reduction in one or more symptoms of the disease under treatment, or that produces a statistically significant improvement in a recognized clinical marker of the disease. Typically, an inhibition effective amount of the chlorite solution will vary between about 0.1 ml/kg to about 1.5 ml/kg, preferably, about 0.5 ml/kg of body weight and at a concentration of about 40 to about 80 mMol ClO₂⁻ per liter, preferably about 60 mMol ClO₂⁻ per liter, respectively. Without being bound by any theory, applicants believe that the relationships described above between the effects on antigen presentation and the clinical results achieved in treating certain diseases means that the therapeutically effective amount will be similar or the same as the inhibition effective amount

Preferably, the chlorite solution of the invention is administered once daily for anywhere from about three to seven days, preferably five days, followed by a period of rest of from 10 to 20 days, preferably from 14-18 days, and more preferably, 16 days, to constitute one cycle of treatment. Preferably, patients are treated with more than one cycle, more preferably, at least three cycles, and most preferably, at least five cycles. The skilled artisan will recognize, however, that other regimens are possible, and may in fact be preferable. Methods of manipulating such regimens are well known in the art.

For example, an alternative treatment regimen consists of intravenously administering the stabilized chlorite solution of the invention once daily for a period of five days, followed by two days of rest (*e.g*., over the weekend), followed by five more consecutive days of administration, followed by a period of rest from anywhere between 1 and 4 weeks to constitute one cycle. Preferably, patients are treated with more than one cycle, more preferably more than three. Skilled artisans are capable of modifying the administration of the stabilized chlorite solution of the invention depending on the disease treated and the size of the patient, using the guidelines provided herein.

The use of an aqueous solution containing a stabilized chlorite solution for treating wounds and infections is known in the art. United States Patent Nos. 4,507,285 and 4,725,437, and EP 0 200 157 describe the use of a stabilized chlorite solution in stimulating the wound healing response in humans, as well as in treating infections caused by parasites, fungi, bacteria, viruses and/or mycoplasma. Kühne *et al*., European Patent No. 200,156, describes the use of a stabilized chlorite solution in conjunction with radiation therapy to aid in repairing damaged irradiated tissue and reducing side effects.

The mode of action in treating damaged and/or infected tissue is thought to involve amplifying the "oxidative burst" response of phagocytes in the presence of bioactivators, e.g., heme compounds. Wound healing and treatment of the reported infections are believed to be effected via activation of macrophages, which in turn serve to activate fibroblast cells that stimulate the wound healing response. The stabilized chlorite solutions are thought to activate macrophages by complexing with the heme moieties present in the macrophage membrane. Upon activation, the macrophages stimulate the fibroblast cells which in turn generate collagen and endothelial cells that are useful in repairing damaged tissue caused by the wound or by the infections.

While not intending on being bound by any theory, the present inventors believe that a macrophage is stimulated by the stabilized chlorite solution by the following sequence of events. In the presence of heme compounds (*e.g.* , hemoglobin, myoglobin, peroxidases, cytochromes, etc.), which are present in the serum which also are part of the cell membrane of phagocytic cells like macrophages, the stabilized chlorite solution becomes a secondary oxidant with oxidative properties different from chlorite and hydrogen peroxide. Indeed, the stabilized chlorite solution of the invention has shown significant pharmacological differences when compared to equimolar chlorite solutions.

The present inventors believe further that the known wound-healing mechanism via macrophage activation of the chlorite solution of the invention also stimulates and enhances the phagocytic activity of the macrophage. Thus, the activated macrophage is primed to ingest, digest and dispose of foreign antigens. The use of a stabilized chlorite solution to render macrophage phagocytic is described in EP 0 200 157.

Prior to the present invention, however, it was not known that a stabilized chlorite solution also can inhibit an antigen-specific immune response, while at the same time enhance the activity of phagocytes. While not intending to be bound by any theory, the present inventors believe that the stabilized chlorite solution, when administered to a mammal in need thereof, partially or completely impedes the antigen presentation of antigen presenting cells (APCs) by activating the alternative macrophage activation pathway. Throughout this description, the expression, "antigen presenting cells" denotes a cell that is capable of presenting an antigen and eliciting an immune response. Useful antigen presenting cells include macrophages and dendritic cells. Inhibition of antigen presentation upon administration of a stabilized chlorite solution is demonstrated by the *in vitro* data described in the examples.

A typical immune response involves stimulating a macrophage, the stimulated macrophages present MHC Class I and II bound antigens on the surface, which, when coupled with the T cell receptor, will stimulate T cells (typically a T cell subset such as CD4 or CD8 cells, and the like) to proliferate and form cytotoxic T-lymphocytes (CTL) cells which in turn kill cells expressing the antigen. After antigen presentation and upon coupling with the T cell receptors, the stimulated APC (macrophage and the like) also secretes various cytokines that can aid in the proliferation of CTLs. Cytokines, or growth factors, are hormone-like peptides produced by diverse cells and are capable of modulating the proliferation, maturation and functional activation of particular cell types. Herein, cytokines refer to a diverse array of growth factors, such as hematopoietic cell growth factors (e.g., erythropoietin, colony stimulating factors and interleukins), nervous system growth factors (e.g., glial growth factor and nerve growth factor), mostly mesenchymal growth factors (e.g., epidermal growth factor), platelet-derived growth factor, and fibroblast growth factor I, II and III, including interferons.

It will be appreciated that there may be several cytokines that are involved in inducing cell differentiation and maturation, and that cytokines may have other biological functions. In the case of IL-1, there may be several forms, such as IL-1-alpha and IL-1-beta, which nevertheless appear to have a similar spectrum of biological activity. Those cytokines that are primarily associated with induction of cell differentiation and maturation of myeloid and possibly other hematopoietic cells include, *inter alia*, IL-1, G-CSF, M-CSF, GM-CSF, Multi-CSF (IL-3), and IL-2( T-cell growth factor, TCGF). IL-1 appears to have its effect mostly on myeloid cells, IL-2 affects mostly T-cells, IL-3 affects multiple lymphocyte precursors, G-CSF affects mostly granulocytes and myeloid cells, M-CSF affects mostly macrophage cells, GM-CSF affects both granulocytes and macrophage. Other growth factors affect immature platelet (thrombocyte) cells, erythroid cells, and the like.

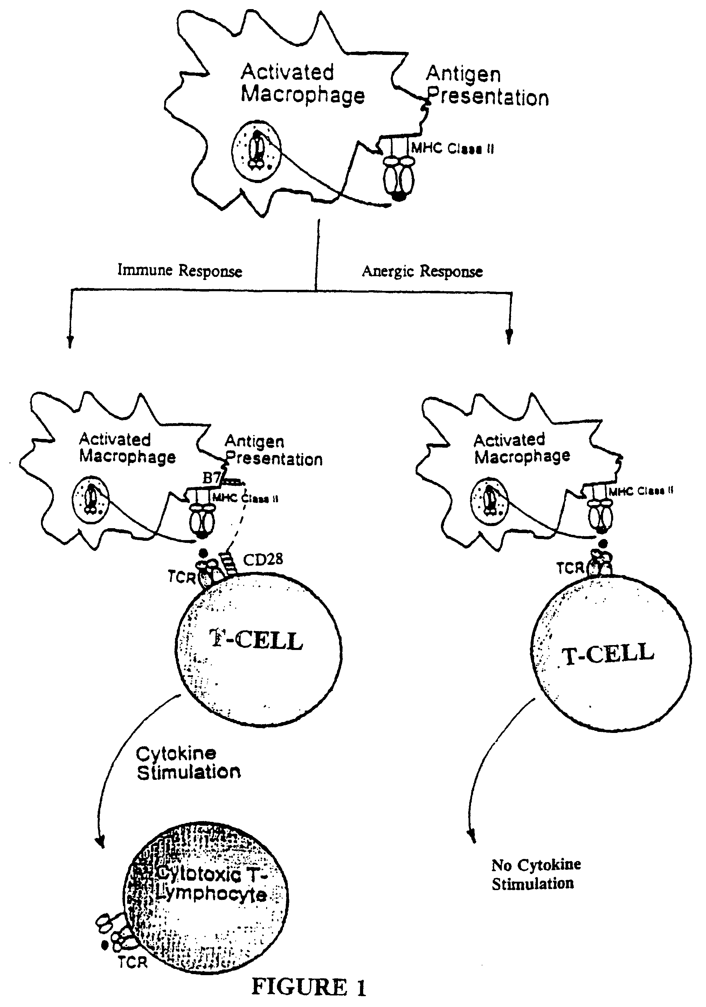

As shown in Figure 1, when an antigen is presented to a patient with a normal, or uncompromised, immune system, the following sequence of events typically takes place. This mechanism can be seen on the left-hand side of Figure 1 labeled "Immune Response." The antigen (or foreign body) is enclosed in vesicles in the macrophage which breaks down the foreign matter into smaller antigenic peptides. An MHC class II molecule transports one of the smaller antigenic peptides to the surface of the macrophage, where it is presented to a T cell receptor (TCR). Binding with the cell receptor triggers the release of activating factors and cytokines such as IL-1, TNF, etc., which restores the self-defense of the macrophage and enhances the intracellular killing of the foreign body. If binding does not occur, the activating factors are not released and the macrophage will not break down the foreign matter into smaller peptides. As it is used in this description, the expression "antigen presentation" therefore denotes the process of presentation of a foreign antigen copied to an MHC Class II molecule on the surface of an APC followed by subsequent binding with a TCR.

As described above, the alternative macrophage activation pathway is thought to act as an important modulator of the proinflammatory macrophage response, and alternatively activated macrophages are thought to play a key role in reducing inflammation in allergic and autoimmune diseases. Without being bound by any theory, the inventors believe that one of the mechanisms by which administration of a stabilized chlorite solution operates to prevent and/or inhibit antigen presentation is by activation of the alternative macrophage activation pathway. Indeed, it is noteworthy that the period of suppression of antigen presentation by a stabilized chlorite solution, which appears to last for periods of says to weeks without the need for further administration of drug, closely parallels the duration of expression of MIP-4 in alternatively activated macrophages, which also remains elevated over an extended period. This extended period of MIP-4 expression indicates that the macrophages also remain activated and can play an anti-inflammatory role over the entire period of activation.

Previously known therapies for preventing T cell proliferation typically acted on cytotoxic T-cells after cytokine stimulation. For example, cyclosporin A is believed to act on the cytotoxic T-Lymphocyte shown at the bottom left of Figure 1 to prevent T-cell proliferation. At this point, however, the APC already has released cytokines that might assist CTL proliferation. Accordingly, a significant amount of these drugs must be administered to prevent the CTL proliferation. There are no known methods for impeding an immune response, however, where the APC or TCR are affected in a manner that partially or completely interrupts the antigen presentation interaction between the APC and the T cell.

Patients suffering from autoimmune diseases and diseases caused by inappropriate immune response such as myasthenia gravis, systemic lupus erythematosus, serum disease, type I diabetes, rheumatoid arthritis, juvenile rheumatoid arthritis, rheumatic fever, Sjörgen syndrome, systemic sclerosis, spondylarthropathies, Lyme disease, sarcoidosis, autoimmune hemolysis, autoimmune hepatitis, autoimmune neutropenia, autoimmune polyglandular disease, autoimmune thyroid disease, multiple sclerosis, inflammatory bowel disease, colitis, Crohn's disease, chronic fatigue syndrome, and the like, do so because the immune response is inappropriate. Chronic obstructive pulmonary disease (COPD) also may have some autoimmune etiology, at least in some patients. In an autoimmune response, the patient's body produces too many CTLs, or other cytokines which turn against the body's own healthy cells and destroy them. In transplant or graft patients, an inappropriate immune response occurs because the immune system recognizes the transplanted organ or graft's antigens as foreign, and hence, destroys them. This results in graft rejection. Likewise, transplant and graft patients can develop a graft vs. host response where the transplanted organ or graft's immune system recognizes the host's antigen as foreign and destroys them. This results in graft vs. host disease. Other inappropriate immune responses are observed in allergic asthma, allergic rhinitis and atopic dermatitis.

In addition, diseases that produce symptoms of chronic inflammation also involve an inappropriate immune response, characterized by excessive macrophage activation. For example, a healthy response to tissue insult, such as a physical wound, or invasion by pathogenic organisms such as bacteria or viruses, involves activation of macrophages (via the "conventional," proinflammatory route) and leads to an inflammatory response. However, this response can "overshoot" in an inappropriate manner, leading to chronic inflammation if the proinflammatory immune response cannot be suppressed. Diseases such as hepatitis B and C , chronic hepatitis, and manifestations of COPD such as obstructive bronchitis and emphysema that apparently are caused by prolonged exposure to non-specific bronchial and pulmonary irritants, are characterized by chronic inflammation (of the liver in hepatitis and of the pulmonary tissue in COPD) induced by excessive macrophage activation.

Conventional therapies for autoimmune diseases such as systemic lupus erythematosus and transplant rejection invoke application of cytotoxic agents, particularly those that affect the lymphoid system (and therein particularly inhibit proliferation of T-lymphocytes). These cytotoxic drugs are similar to those often used in cancer chemotherapy, and have well known myeloid and other hematopoietic side effects. In addition to these drugs, specific antibodies against lymphoid cells, particularly T-cells, have been used as immunosuppressive agents. For example, Uchiyama *et al*., ( *J. Immunol.* 126:1393 and 1398 (1981)) described an anti-Tac monoclonal antibody that specifically binds the human IL-2 receptor of activated T-cells, and which can be conjugated to cytotoxic agents, such as drugs, toxins or radioisotopes, to effect a relatively select killing of these cells involved in organ rejection. Such antibodies can be conjugated with a β- or α-emitting radioisotope, and can be administered to a patient prior to undertaking organ transplantation and, if needed, also thereafter. The aqueous solution containing a stabilized chlorite solution can be used in place of the aforementioned agents. Alternatively, stabilized chlorite solution can be used in combination with the conventional immunosuppressive agents.

Administering an aqueous solution containing a stabilized chlorite solution to a mammal inhibits the antigen-specific immune response without compromising the immune system entirely, because the solution also is effective in enhancing phagocytic activity. Thus, the present invention encompasses methods of treating auto-immune diseases, preventing transplant organ or graft rejection and septic shock as a result thereof, and reducing inappropriate immune responses such as excessive inflammation and allergic reaction. Because other methods already are known to treat these disorders, skilled artisans are capable of modifying the known techniques by administering an inhibition effective amount of an aqueous solution containing a stabilized chlorite solution, using the guidelines provided herein. For example, skilled artisans are capable of designing a treatment regimen to treat any of the aforementioned disorders using the stabilized chlorite solution of the invention by varying the dosage amount, frequency of administration, or mode of administration.

A preferred embodiment of the treatment of this invention entails administration to a mammal in need thereof, an aqueous solution of a product that has been termed "tetrachlorodecaoxygen anion complex," commonly abbreviated as "TCDO." This substance can be prepared using the procedures described in Example 1 of U.S. Patent No. 4,507,285 ("the '285 patent"), and is a water clear liquid, miscible with alcohols, and has a melting point of -3°C. The Raman spectrum shows bands of 403, 802 (chlorite) and 1562 cm⁻¹ (activated oxygen). The skilled artisan will recognize that any chemically stabilized chlorite solution can be used in the methods of the present invention, and that the scope of the invention is not limited to use of the product described in the '285 patent.

The present invention, thus generally described, will be understood more readily by reference to the following examples, which are provided by way of illustration and are not intended to be limiting of the present invention. In the examples, "WF10" denotes an aqueous stabilized chlorite solution.

### Example 1

In this example, and the following examples 2-4, details regarding the methods used in performing these examples can be found in *Fagnoni et al*., *Immunology*, **85:** 467-74 (1995). This example, together with the following examples 2-4, elucidate the role of a stabilized chlorite solution in preventing dendritic cell-mediated costimulation..

### Effect of WF10 on Dendritic Cell (DC) Stimulated Allogeneic MLR

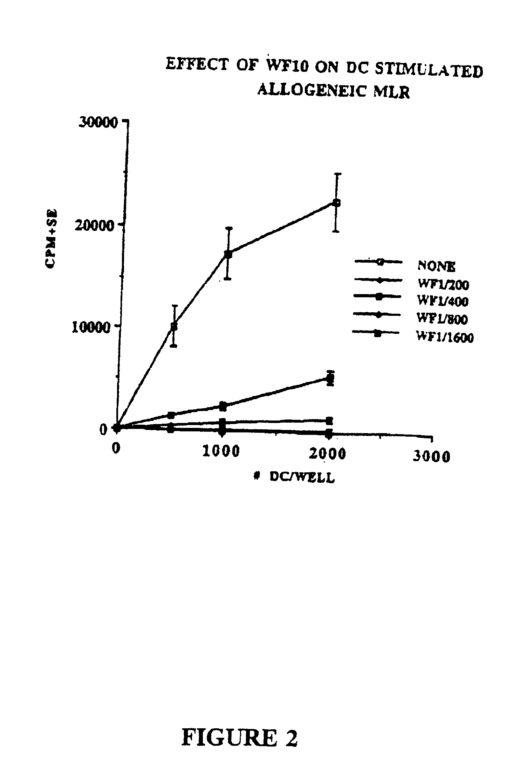

Dendritic cells, T cells and monocytes were obtained in the manner described in Fagnoni *et al*. To assess the effects of WF10 on DC-dependent T cell activation, freshly isolated CD4⁺-T cells were activated with allogeneic MLR in the presence or absence of WF10 to DC. Purified resting CD4⁺ T cells (5-10 × 10⁴/well) were cultured with irradiated (25 Gy) allogeneic DC in U-bottomed 96-well plates containing 200 µl of complete medium. The cultures were maintained at 37°, 8% CO₂ in humidified air for 5 days. Cultures were pulsed with 1 µCi [³H]thymidine (6-7 Ci/mm, New England Nuclear, Boston MA) 19 hour before harvest. The [³H]thymidine incorporation by proliferating cells was measured in a β-scintillation counter. WF10 was added to DC stimulated allo-MLR DC and incubated at 4° for about 3 minutes before the addition of CD4⁺ T cells. The number of proliferated T-cells for samples using no WF10, and for samples using WF10 are shown in Figure 2. The results in Figure 2 represent the mean ± SEM of qudruplicate cultures, and data are representative of four experiments

As shown in Figure 2, the CD4⁺ T cell response to DC stimulated allogenic MLR was inhibited in a dose-dependent manner by WF10. The WF10 was administered by adding WF10 to culture medium at time 0 in doses of 25 µg/ml or 50 µg/ml. As seen in Figure 2, even as the number of dendritic cells (DC) per well was increased, the number of CPM + SE (counts per minute + standard error) remained essentially the same, with the greatest degree of inhibition resulting from WF10/1600. The expression WF10/number denotes that dilution of WF10 and designates the amount of WF10 per ml of solution. For example, WF10/1600 denotes a diluted solution of WF10 containing 1 ml of WF10 per 1600 ml of solution.

### Example 2

### Effect of WF10 on Monocytes Stimulated Allogeneic MLR

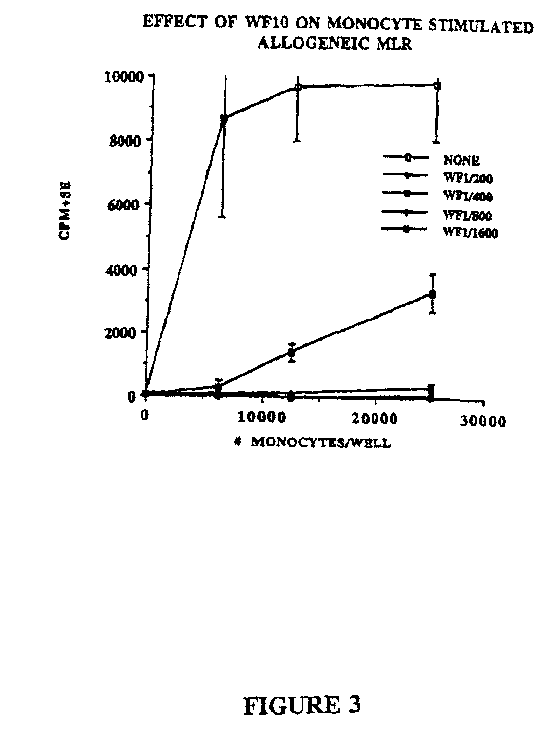

Example 1 was repeated with the exception that adherent monocytes, obtained in accordance with Fagnoni *et al*. Were used instead of DC. The results are shown in Figure 3, and demonstrate that administration of WF10 was effective in inhibiting proliferation of CD4⁺ T-cells from monocyte stimulated MLR. Indeed, with administration of WF1/1600, the stabilized chlorite solution was effective in completely inhibiting proliferation of CD4⁺ T-cells from monocytes stimulated with allogeneic MLR, despite increased concentration of monocytes per well.

The results of examples 1 and 2 therefore show that WF10 is effective in inhibiting proliferation of CD4⁺ T cells from DC or monocytes stimulated with allogeneic MLR.

### Example 3

Examples 3 and 4 were carried out to determine the effect of WF10 on the inhibition of antigen-induced proliferation of T cells using various antigens. In this example, purified resting CD4⁺ T cells (5-10 × 10⁴/well) were cultured with irradiated (25 Gy) autologous DC in U-bottomed 96-well plates containing 200 µl of complete medium. The cultures were maintained at 37°, 8% CO₂ in humidified air for 6 days. Cultures were pulsed with 1 µCi [³H]thymidine (6-7 Ci/mm, New England Nuclear, Boston MA) 19 hours before harvest. The [³H]thymidine incorporation by proliferating cells was measured in a β-scintillation counter.

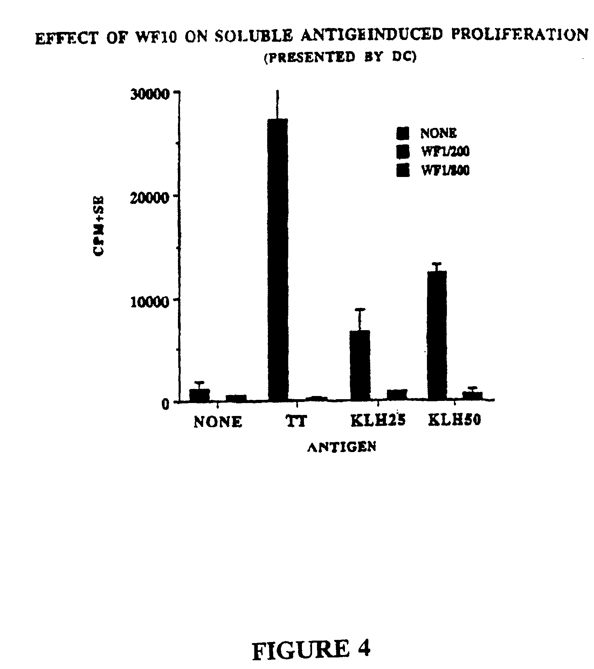

Soluble keyhole limpet hemocyanin (KLH) and tetanus toxoid (TT) were added to autologous DC. Measurements were taken for no addition of WF10, addition of WF10/200 and WF10/800 (representing administration of WF10 to the culture medium at time 0 of 0, 1ml/200 ml of solution and 1 ml/800 ml of solution, respectively) to determine the proliferation of CD4⁺ T cells when no antigen, TT, KLH25 (25 µg/ml) and KLH50 (50 µg/ml) were presented by DC. The number of proliferated T-cells for samples using no WF10, and for samples using WF10 are shown in Figure 4. The results in Figure 4 represent the mean ± SEM of qudruplicate cultures, and data are representative of four experiments.

As shown in Figure 4, significant proliferation of CD4⁺ T cells occurred when DC presented the soluble antigens KLH and TT. Administration of WF10, however, almost completely inhibited the proliferation of CD4⁺ T cells when either KLH or TT were presented by DC.

### Example 4

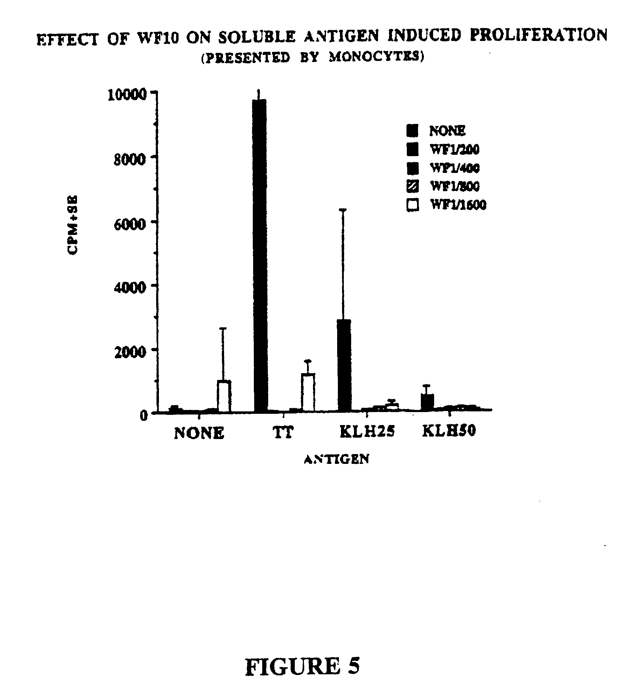

Example 3 was repeated except that monocytes were used instead of DC for antigen presentation. In addition, WF10 was administered in the following increments WF10/200, WF10/400, WF10/800 and WF10/1600. The results are shown in Figure 5. As shown in Figure 5, there was significant proliferation of CD4⁺ T cells when monocytes presented the soluble antigens KLH and TT. Administration of WF10, however, almost completely inhibited the proliferation of CD4⁺ T cells when either KLH or TT were presented by monocytes.

The results achieved by administration of an aqueous solution containing a stabilized chlorite solution reveal that it is capable of inhibiting an antigen-specific immune response. It has previously been reported that administration of an aqueous solution containing a stabilized chlorite solution is effective in enhancing phagocytic activity. Thus, it now is possible by administering only one medicament to inhibit one type of immune response, (antigen presentation and proliferation of T cells) while at the same time, enhance another type of immune response (phagocytosis).

### Example 5

A phase 2 trial was conducted at San Francisco General Hospital. The study enrolled 18 patients in an open label pathogenesis study of WF-10. Patients received one hour infusions of WF-10 for one week, followed by two weeks of rest. On the third week, the patients again received one hour infusions of WF-10 daily for one week followed by two weeks of rest. Parameters studied included measures of macrophage activation/function immunologic activation and HIV viral load. RBC hemolysis evaluation studies included 51 Cr-RBC survival studies compared with changes in hemoglobin, haptoglobin and reticulocyte values.

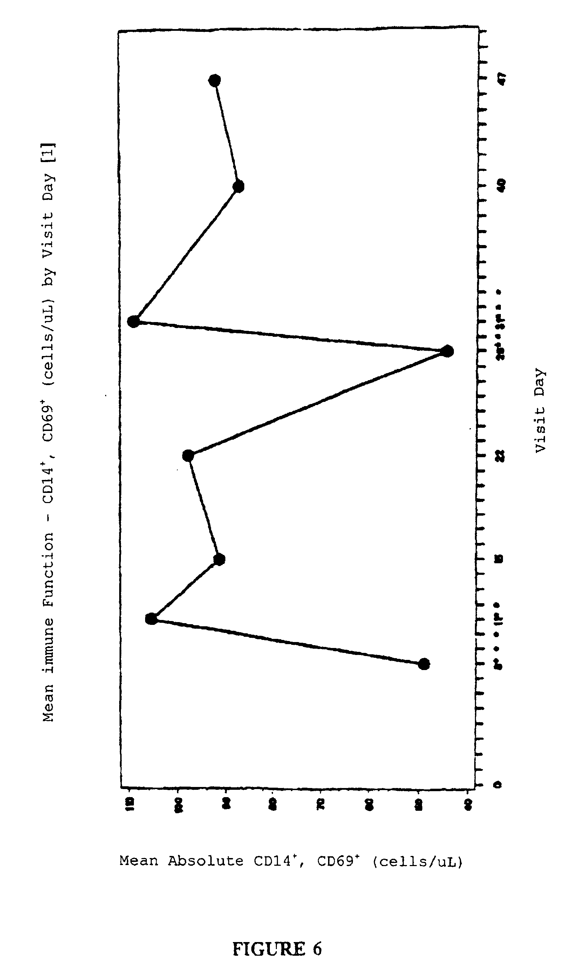

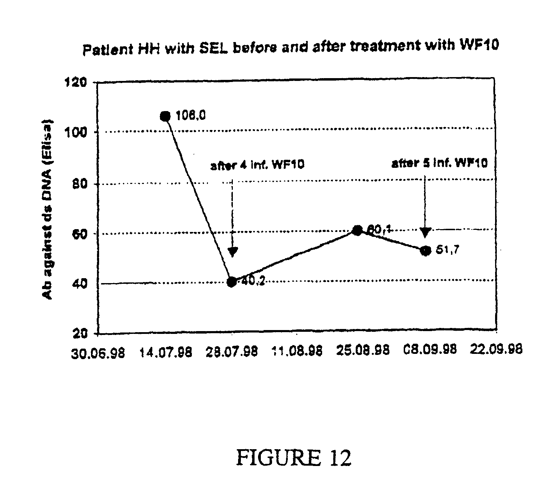

There were no side effects noted in any of the 18 patients. Data on eight of the patients were gathered and the results are tabulated below, and depicted in Figures 6-13. There appeared to be acute increases in the following parameters as measured by flow cytometry (FACSCAN as recommended by, for example, Becton-Dickinson) in relation to drug administration, changes that generally returned close to baseline within 2 weeks of drug administration: CD-4, CD-8, CD14⁺/CD69⁺, CD14⁺ side scatter, CD20/DR⁺ cells. Several values seemed to generally increase through the study, showing no clear downward trend by the end of the study and may represent long-term changes induced by WF-10. These include an increase in macrophage phagocytosis index and an increase in the CD3⁺/CD8⁺/CD28⁻ subset of T-cells.

Potential downward trends were noted in the following categories: macrophage intracellular TNF-α secretion, and a decrease in the number of circulating CD14⁺/DR⁺ cells. It has been reported that immune paralysis results when the number of circulating CD14⁺/DR⁺ cells decreases to such an extent as to reach a threshold value. No obvious changes were noted in T-cell PHA activation values or HIV load as measured by the HIV bDNA assay (most of the patients had no detectable HIV thoughout the study). Results of the RBC survival studies showed no evidence for hemolysis in response to the treatment.

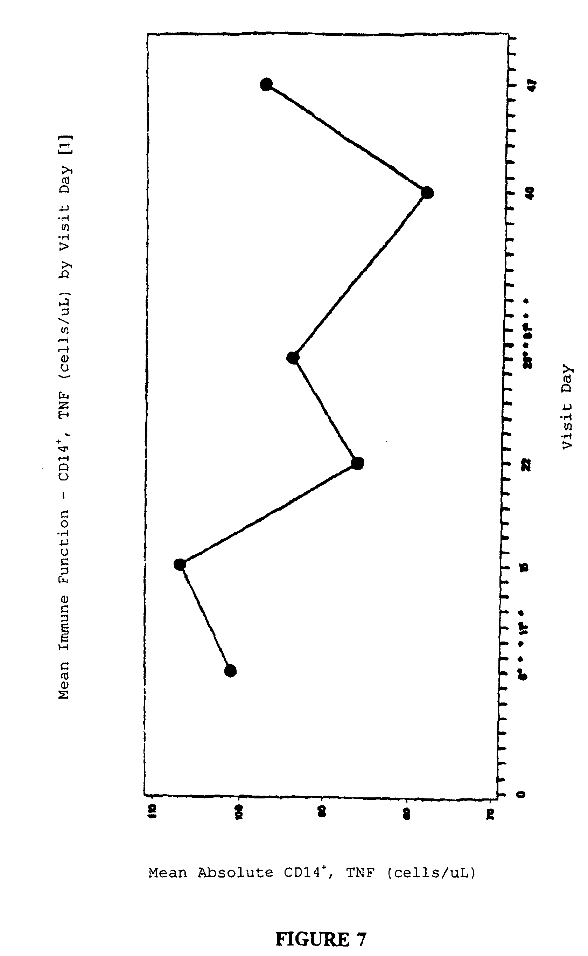

As shown in Figure 6, administration of WF10 results in an increase in CD14⁺/CD69⁺ cells, with dramatic increases immediately following infusion. Figure 7 shows a decrease in CD14⁺/TNF secretion after administration of WF10, thereby indicating that a stabilized chlorite solution is effective in decreasing secretion of the tumor necrosis factor cytokine.

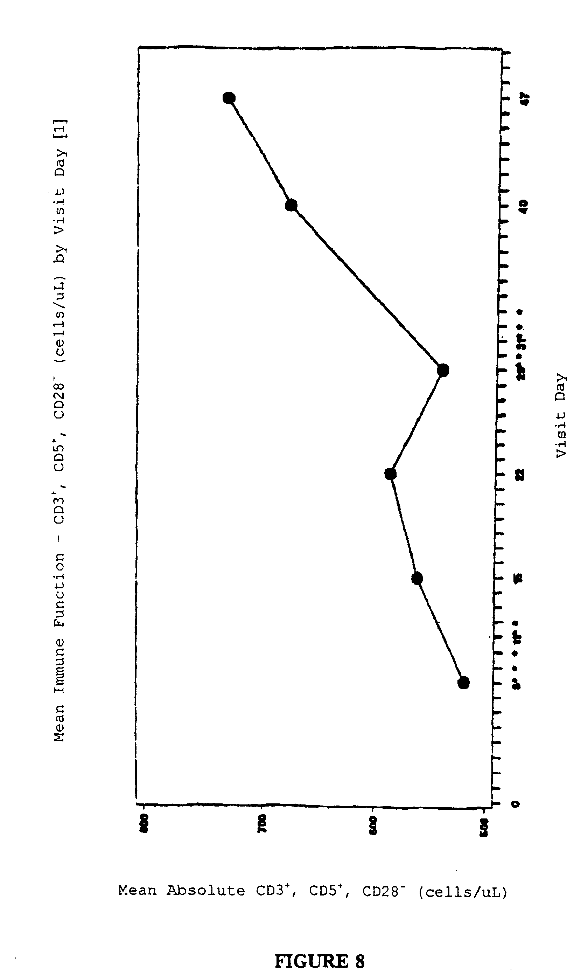

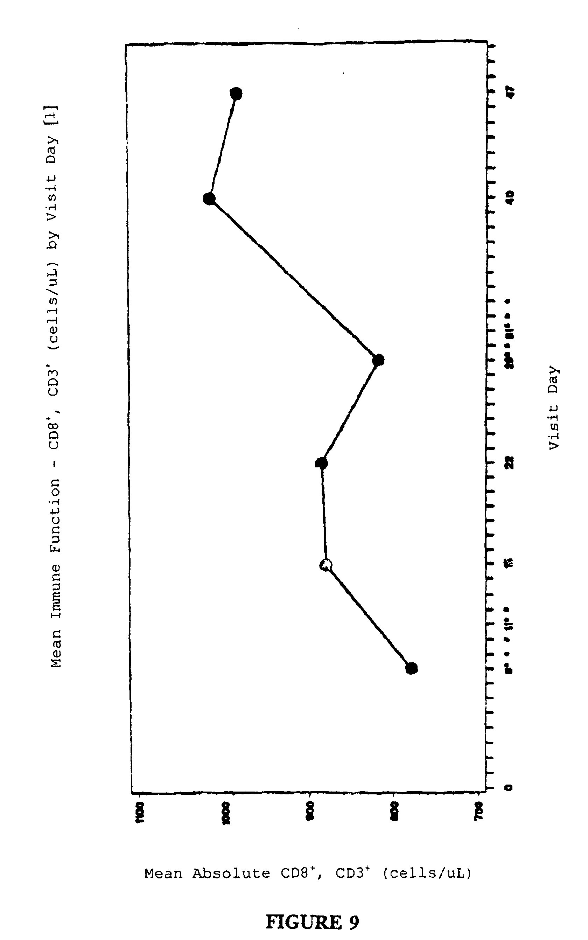

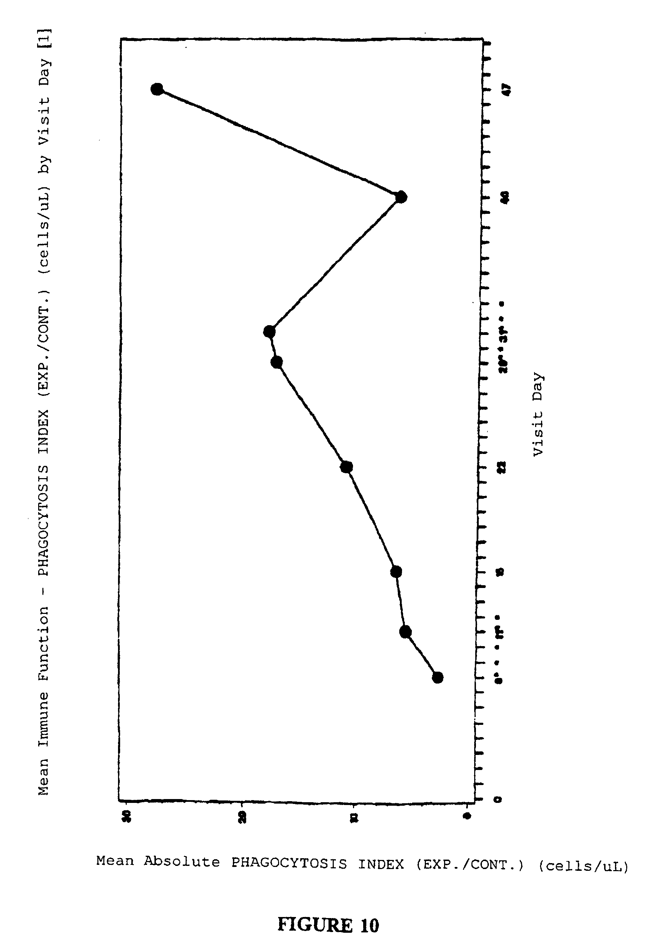

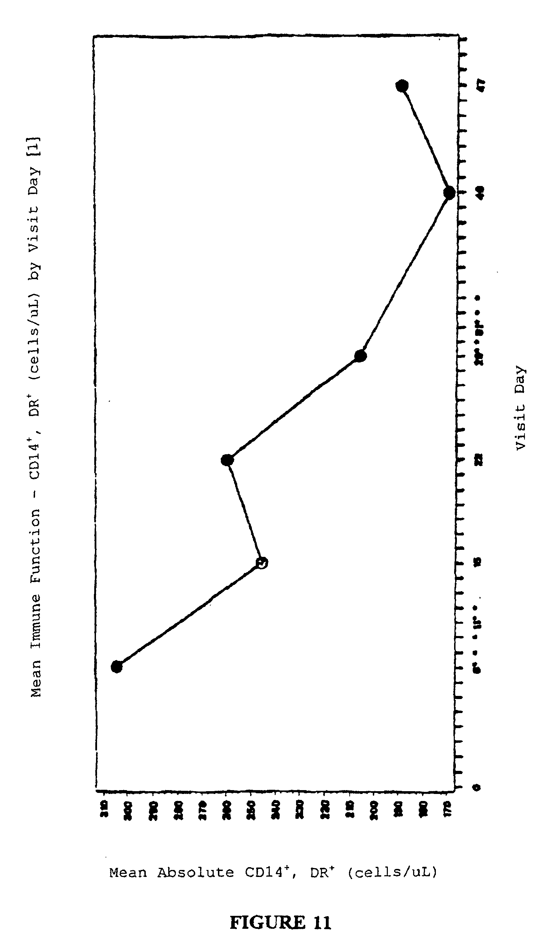

Figures 8 and 9 show that administration of WF10 to patients *in vivo* results in a steady increase in the number of CD3 ⁺/CD8⁺, as well as a steady increase in the number of CD3⁺/CD8⁺/CD28⁻ T cells. The *in vitro* data above show inhibition of antigen presentation using CD4⁺ T cells, and Figures 8 and 9 show an increase in the number of circulating CD28⁻ T cells (CD3⁺ T cells). Figure 10 illustrates an increase in phagocytosis index upon administration of WF10. Figure 11 shows a decrease in immune function upon administration of WF10 by virtue of the decrease in CD14⁺/DR⁺ cells. The inventors therefore believe that the stabilized chlorite solution of the invention is capable of up-regulating phagocytosis, while at the same time, down-regulating or suppressing the cell-mediated and humoral immune response.

The results tabulated below summarize the data from 15 patients and show the changes in various measured parameters between the 8^{th} day and the 47^{th} day of treatment. The 8^{th} day represents the first day of WF10 administration because the first 7 days of treatment are devoted to patient evaluation.

| **Parameter Measured** | **p-value*** | **Direction** |
|---|---|---|
| CD3⁺, CD8⁺, CD28⁻ | 0.027 | increase |
| CD14⁺, TNF- | 0.017 | decrease |
| CD14⁺, DR⁺ | 0.032 | decrease |
| CD3⁺, CD4⁺, CD38⁺ (MF CD38 Antigen) | 0.021 | decrease |
| CD3⁺, CD8⁺, CD28⁺ (MF CD28 Antigen) | 0.010 | decrease |
| CD20⁺, DR⁺ (MF DR Antigen) | 0.014 | decrease |
| All CD14⁺ | 0.037 | decrease |

| | | |
|---|---|---|
| * - One-tailed p-value. Sample size of 15 patients using Wilcoxon rank statistic. | | |

These data show that administration of WF10 *in vivo* to humans shows an increase in the production of CD28⁻ subset of CD8⁺ T-cells. The data also show an increase in macrophage activation leading to phagocytosis. The data further show no evidence of RBC hemolysis. When coupled with the *in vitro* studies showing the inhibition of antigen presentation for CD4⁺ cells, it is believed that administration of WF-10 *in vivo* will result in inhibition and/or prevention of antigen presentation in APC, as well as stimulate macrophage activation resulting in increased phagocytosis.

### Example 6

Based on the *in vivo* data above, administration of WF10 has shown a consistent down regulation of CD14⁺/DR⁺ cells achieving statistical significance. In addition, WF10 administration *in vivo* has shown overall reduction of CD3⁺/CD8⁺/CD28⁺ cells, and significant increased levels of CD3⁺/CD8⁺/CD28⁻ cells of long-term duration. The *in vitro* data above also show that WF10 is effective in inhibiting and/or preventing antigen presentation. This reduced antigen presentation may be critical in inhibiting lymphoproliferative disease, and in particular in inhibiting B-cell lymphoma and thus, it is expected that WF10 therapy will be effective for treatment of lymphoma. In accordance with this expectation, in the case of a single patient suffering from B-cell lymphoma, the patient responded to WF10 therapy with a notable reduction of tumor size with no recurrence to date.

Adult patients having low grade follicular lymphoma are selected based on their lack of enrollment in current therapy regimens. Fifteen patients having lymph nodes > 1 cm in diameter at baseline confirmed by CT scan will be enrolled in an open-label, single arm, single center study. Patients will receive periodic 0.5 ml/kg infusions of WF10 from days 1-5 (week 1) and days 8-12 (week 2). After screening evaluations are completed (about 14 days), eligible patients will attend pre-study visit in week 0 to acquire the baseline data.
Screening criteria include the following:
male or female patients greater than 18 years of age;
histologically confirmed follicular lymphoma;
measurable disease defined as having lymph nodes > 1 cm in diameter as measured by CT;
adequate renal function documented by a serum creatinine < 2 times in institution's ULN;
adequate liver function documented by a serum billrubin less than or equal to 1.5 mg/dl and SGOT (AST) or SGPT (ALT) < 5 times the institutional upper limit of normal;
written informed consent to participate in this study and a willingness to comply with all procedures and scheduled visits;
hemoglobin > 9.0 g/dl for woman and > 10.0 g/dl for men;
platelet count > 75,000/mm²; and
absolute neutrophil count > 750/mm².

WF10 will be applied at a dose of 0.5 ml per kg of body weight diluted into 250 to 500 ml normal saline administered by intravenous infusion of 1 hour duration. CT measurements will be taken to determine tumor size at week 0, on day 15, day 30 and day 45. Follow-up period will last for a duration of 3 months with final CT measurements on day 90.

CT measurements reveal that administration of WF10 results in a reduction of lymph node size. Patients also exhibit an increase in CD3 ⁺/CD8⁺/CD28⁻, an increase in CD14⁺/DR⁺ and an increase in CD40 T cell subsets.

While the invention has been described in detail with reference to the examples and particularly preferred embodiments, those skilled in the art will appreciate that various modifications can be made to the invention without departing from the spirit and scope thereof. All documents referred to above are incorporated by reference. The specification of United States Provisional Application 60/060,953, filed October 6, 1997, for which benefit under 35 USC § 119 is claimed, is expressly incorporated by reference in its entirety.

## Claims

1. Use of a stabilized chlorite solution for the preparation of a medicament, comprising an inhibition effective amount of said solution, for the treatment of conditions associated with inappropriate or excessive antigen presentation in a mammal, whereby an antigen-specific immune response is inhibited by way of activation of an anergic response.

2. Use according to claim 1, wherein the antigen presenting cells are dendritic cells or macrophages.

3. Use according to claim 1, wherein the antigen-specific immune response is an antigen receptor-induced T cell response.

4. Use according to any of claims 1 to 3 for the treatment of an autoimmune disease.

5. Use according to claim 4, wherein said disease is selected from the group consisting of myasthenia gravis, systemic lupus erythematosus, serum disease, type I diabetes, rheumatoid arthritis, juvenile rheumatoid arthritis, rheumatic fever, Sjörgen syndrome, systemic sclerosis, spondylarthropathies, Lyme disease, sarcoidosis, autoimmune hemolysis, autoimmune hepatitis, autoimmune neutropenia, autoimmune polyglandular disease, autoimmune thyroid disease, multiple sclerosis, inflammatory bowel disease, colitis, Crohn's disease, and chronic fatigue syndrome.

6. Use according to any of claims 1 to 3, wherein the condition is organ transplant or graft rejection.

7. Use according to any of claims 1 to 3, whereby the condition is inflammation.

8. Use according to claim 7, wherein said condition is a disease selected from the group consisting of hepatitis B, hepatitis C, chronic hepatitis, and chronic obstructive pulmonary disease.

9. Use according to claim 1, wherein said condition is a lymphoproliferative disease.

10. Use according to claim 9, wherein said lymphoproliferative disease is lymphoma.

## Patentansprüche

1. Verwendung einer stabilisierten Chloritlösung für die Herstellung eines Medikamentes, enthalten, eine inhibitorisch wirksame Menge dieser Lösung, für die Behandlung von Zuständen, die mit einer unangemessenen (ineffizienten, falschen) oder übermäßigen Antigenpräsentation in einem Säuger verbunden sind, wobei eine antigenspezifische Immunantwort über die Aktivierung einer anergischen Antwort inhibiert wird.

2. Verwendung nach Anspruch 1, worin die Antigen präsentierenden Zellen dendritische Zellen oder Macrophagen sind.

3. Verwendung nach Anspruch 1, worin die antigenspezifische Immunantwort eine über den Antigenrezeptor injizierte T-Zell-Antwort ist.

4. Verwendung nach einem der Ansprüche 1 bis 3 für die Behandlung einer Autoimmunerkrankung.

5. Verwendung nach Anspruch 4, worin die genannte Erkrankung aus der Gruppe ausgewählt ist, die aus Myasthenia gravis, systemischem Lupus erythematodes, Serumkrankheit, Typ I Diabetes, rheumatoider Arthritis, juveniler rheumatoider Arthrits, rheumatischem Fieber, Sjögren Syndrom, systemischer Sklerose, Spondylarthropatien, Lyme-Krankheit, Sarkoidose, autoimmuner Hämolyse, autoimmuner Hepatitis, autoimmuner Neutropenie, autoimmuner polyglandulärer oder pluriglandulärer Erkrankung, autoimmuner Schilddrüsenerkrankung, Multipler Sklerose, entzündlicher Darmerkrankung, Kolitis, Morbus Crohn und chronischem Ermündungssyndrom besthet.

6. Verwendung nach einem Ansprüche 1 bis 3, worin der Zustand einer Organtransplantation oder eine Implantat- oder Transplantat-Abstoßung ist.

7. Verwendung nach einem der Ansprüche 1 bis 3, worin der Zustand eine Entzündung ist.

8. Verwendung nach Anspruch 7, worin der Zustand eine Erkrankung ist, die aus der Hepatitis B, Hepatitis C, chronischer Hepatitis und chronischer obstruktiver Atemwegserkrankung bestehenden Gruppe ausgewählt ist.

9. Verwendung nach Anspruch 1, worin der Zustand eine lymphopoliferative Erkrankung ist.

10. Verwendung nach Anspruch 9, worin die lymphopoliferative Erkrankung ein Lymphom ist.

## Revendications

1. Utilisation d'une solution stabilisée de chlorite pour la préparation d'un médicament comprenant une quantité de la solution efficace pour une inhibition, pour le traitement d'états associés à une présentation inappropriée ou excessive d'antigènes chez le mammifère, une réponse immunitaire spécifique de l'antigène étant inhibée au moyen de l'activation d'une réponse anergique.

2. Utilisation suivant la revendication 1, dans laquelle les cellules présentant l'antigène sont des cellules dendritiques ou des macrophages.

3. Utilisation suivant la revendication 1, dans laquelle la réponse immunitaire est induite par un récepteur d'antigène.

4. Utilisation suivant l'une quelconque des revendications 1 à 3 pour le traitement d'une maladie autoimmunite.

5. Utilisation suivant la revendication 4, dans laquelle la maladie est choisie dans le groupe consistant en la myasthénie grave, le lupus érythèmateux aigu disséminé, la maladie du sérum, les diabètes de type I, la polyarthrite chronique évolutive, la polyarthrite juvénile, le rhumatisme aigu, le synchrome de Sjörgen, la sclérose systémique, les spondylarthropaties, la maladie de Lyme, la sarcoïdose, l'hémolyse autoimmune, l'hépatite autoimmune, la neutropenie autoimmune, la maladie polyglandulaire autoimmune, la maladie de la thyroïde autoimmune, la sclérose en plaques, les infections inflammatoires du tube digestif, la colite, la maladie de Crohn et le syndrome de fatigue chronique.

6. Utilisation suivant l'une quelconque des revendications 1 à 3, dans lequel l'état est une transplantation d'organes ou un rejet de greffe.

7. Utilisation suivant l'une quelconque des revendications 1 à 3, dans le cas l'état est une inflammation.

8. Utilisation suivant la revendication 7 dans lequel l'état est une maladie choisie dans le groupe consistant en l'hépatite B, en l'hépatite C, en l'hépatite chronique et en la bronchopneumopathie chronique obstructive.

9. Utilisation suivant la revendication 1, dans lequel l'état est une maladie lymphoproliférative.

10. Utilisation suivant la revendication 9, dans laquelle la maladie lymphoproliférative est le lymphome.
